# EUROPEAN PATENT SPECIFICATION

(11) **EP 0 617 606 B1**
(45) Date of publication and mention of the grant of the patent: **13.03.1996**
(21) Application number: 92924232.9
(22) Date of filing: 04.11.1992
(51) Int. Cl.: A61F 13/58

(54) **TAPE LAMINATES FOR DIAPER CLOSURE**
MEHRSCHICHTIGE VERSCHLUSSBÄNDER FÜR WINDELN
BANDES STRATIFIEES POUR FERMETURE DE COUCHES

(30) Priority: 17.12.1991 GB 9126781
(43) Date of publication of application: 05.10.1994
(73) Proprietor: MINNESOTA MINING AND MANUFACTURING COMPANY, St. Paul, Minnesota 55133-3427 (US)
(72) Inventor: LLOYD, Peter, M., Swansea SA4 1GD (GB)
(74) Representative: Bowman, Paul Alan
(86) International application number: US9209424
(87) International publication number: WO9311728

(56) References cited:
- EP-A- 0 257 752
- US-A- 4 801 480
- US-A- 5 061 535

## Description

### Technical Field

This invention relates to tape laminates and in particular to composite pre-laminated tapes for forming closures, e.g., for disposable diapers.

### Background and Field of the Invention

At least as early as 1955, it had been suggested to use strips of normally tacky and pressure-sensitive adhesive tape to secure conventional cloth diapers on an infant, see for example U.S. Patent Nos. 2,714,889 and 3,221,738. A few years later, when disposable diapers became extremely popular, strips of pressure-sensitive adhesive tape were again employed as closures, see for example U.S. Patent No. 3,620,217.

A disposable diaper typically has a thin, flexible, low density polyethylene film cover, an absorbent filler within the cover, and a porous inner liner overlying the filler. The diaper is positioned at the crotch of an infant with the two ends of the diaper extending toward the front and back, respectively. Edges on each side of the diaper are then either positioned adjacent to each other or overlapped using a strip of pressure-sensitive adhesive tape being adhered to the cover film at border regions adjacent each of the two edges to hold the diaper closed.

After a tape closure has been opened, it is frequently discovered that the diaper has not been soiled and hence that there is no need to replace it. If the diaper cover has not been torn, a second strip of tape can sometimes be applied as a replacement closure, but this is often inconvenient. As a result, considerable work has been undertaken to develop a tape diaper closure that is not only capable of bonding firmly to the diaper cover but is also capable of being opened without destroying the tape diaper closure or the diaper cover and subsequently reclosed. Closures of this type have involved a combination of two or more tapes, one of which remains permanently adhered to one edge of the diaper and the other being removably adhered to the other edge of the diaper. Examples of such products are disclosed in U.S. Patent Nos. 3,951,149, 3,987,793, 3,999,546, 4,020,842, 4,227,530, 4,726,971 and 4,801,480, and European Patent No. 0148587A.

Typically, tape closures for diapers are fabricated by positionably mounting a plurality of individual rolls of the appropriate tapes and combining then in situ to form a composite strip of tape, the width of which is substantially the same as the length of the diaper closure to be fabricated. The composite roll is then severed at right angles to the edges of the composite strip at intervals corresponding to the width of the desired tape closure and adhered at an appropriate location along the border adjacent the side edges of the diaper as exemplified in U.S. Patent Nos. 3,616,114, 4,726,971 and 4,801,480, and European Patent no. 0148487A. Although this manufacturing process is effective, relatively sophisticated machinery is necessary to accomplish the superimposition of several rolls of tape to form a composite strip of tape in situ. Thus, it is desirable to provide diaper manufacturers with a composite pre-laminated tape in a single roll from which tape closures may readily be prepared.

A common feature of such tape closure systems is that the fastening or closure tape comprises a backing, such as paper, polymeric film etc., having an adhesive layer on one side and a coating of a suitable release agent on the other side to facilitate unwinding of the composite tape when wound upon itself about a core. Generally, the backing comprises paper which is coated with a barrier layer prior to application of the release agent in the form of a solution or water-based formulation. The barrier layer prevents the formulation of release agent being absorbed by the porous paper prior to drying thereby ensuring the release agent remains at the surface where it is most effective. Suitable materials for the barrier layer include polyethylene, typical release agents are based on silicone chemistry.

Solventless, radiation-curable release coating compositions are known and are disclosed, for example, in British patent No. 2010698. The compositions may be applied and cured on any suitable substrate including paper, wood, metals, plastics materials, ceramics, glass, concrete etc. The Examples illustrate the release coating being applied to 40 pound (18kg) super-calendared kraft paper.

European Patent Application No. 0315297 discloses that in order to employ radiation-curable silicone coatings on porous and lightly moisture-absorbent paper substrates it is necessary to employ a barrier coating of radiation-insensitive hot melt material e.g a wax-like material. The use of the hot melt wax-like material allows poor grade, porous papers to be successfully coated with radiation-curable silicone and pressure-sensitive adhesive tapes may be formed from such release coated paper.

It has now been found that pressure-sensitive adhesive tapes suitable for use in closure systems may comprise a backing of paper coated directly with a solventless radiation-cured release coating.

Therefore, according to the present invention, there is provided a laminate tape construction comprising two or more tapes each having a layer of pressure-sensitive adhesive on a first face in which at least one of the tapes comprises a porous paper backing having, on the opposite side to said adhesive layer, a continuous release coating of a solventless, radiation-cured silicone applied directly to the paper such that silicone is not on the porous paper backing first face.

The laminate tape construction of the invention is suitable for closure system, particularly for diapers, with at least the fastening tape having the radiation-cured release coating.

The application of the release coating directly to the paper backing has several advantages, including:
1. lower cost backings may be used because of the elimination of one manufacturing process, film extrusion or lamination of the barrier layer and its associated waste.
2. improved degradability due to the elimination of the barrier layer, such as polyethylene, allowing direct access to the paper fibres through the silicone alone.
3. eliminates or reduces need for pretreatment by corona discharge of the paper prior to silicone coating.
4. eliminates the risk of product failure by delamination at the paper barrier layer interface.

The paper backing provides the basic strength of the tape. A clupak paper provides the combination of strength, extensibility or conformability and reasonable tear resistance to suit the needs of the application. The lamination or extrusion of a barrier layer e.g. polyethylene or similar polymer film, to the surface provides solely a non-porous surface for subsequent coatings. It does not materially affect the properties described above.

A suitable paper will typically be bleached for the aesthetic requirements of a hygiene application but an unbleached (brown), semi-bleached (beige or buff) or colored paper would also be functional.

A range of paper basis weight of 50 to 150 g/m² is suitable, typically 80 to 110 g/m².

The elimination of the barrier layer on the paper requires that the release coating laid down must be cured and thus non-migratory in a very short time, before any significant penetration into the paper can occur.

In accordance with the invention, the curing of the solventless silicone begins essentially immediately. If the cure were thermally actuated, a period of 5 to 60 seconds could be necessary to achieve full cross-linking or cure. If, however, the cure is radiation activated, either by electron beam or ultra violet radiation, the curing process will proceed in a fraction of a second. The advantage of this is that the silicone coating will not have a chance to effectively penetrate the paper before being fixed by the cure mechanism. Higher cure rates permit higher run speeds which in turn means the coated paper is transported to the cure module faster thus further reducing the opportunity for absorption. Typical silicone systems are those commercially available from Goldschmidt, Essen of Germany; General Electric of U.S.A; Wacker Chemie of Germany and Dow Corning of U.S.A.

The coating weight of the silicone fluid should be in the range of 0.4 to 2.0 g/m² to provide a continuous coating that satisfactorily permits the subsequent unwinding of a roll of tape whose adhesive is directly in contact with the silicone coating on the lap below.

The coating will be such that when one layer of the finished tape is superimposed upon another, rolled down with a 2kg or similar weight and then the upper tape layer is peeled away at 180 degrees, the force required shall be between 0.1 and 3.0 newtons per 25mm width.

The base paper, as supplied in roll form, may be printed with either a full coating to produce a colored surface or with a definitive legend to identify it or its use. Appropriate printing methods include flexography or rotogravure utilizing commercially available water, solvent-based or solventless inks. Subsequently, the printed or unprinted paper may be corona treated if desired to pretreat the surface to be silicone coated.

The silicone coating may then be applied by commercially available coating stations using the principle of multi-roll gravure coating to achieve the low coating weights required. A typical coating station would use from 3 to 6 coating rolls.

As close as possible to the last nip (the coating nip) of the silicone coating station should be the radiation curing station. This station comprises a source of radiation, typically either ultraviolet or electron beam, directed towards the coated web travelling past. In the event of UV-curing, it may be necessary to also have an inert gas atmosphere above the surface to prevent inhibition of cure by atmospheric oxygen. Typically, this atmosphere is nitrogen.

Subsequently, the siliconized paper may be coated with an adhesive mass either by the application of a hot melt adhesive and subsequent cooling or by the application of a solvent based adhesive of sufficiently high solids content and viscosity that the adhesive does not penetrate the paper surface to any significant degree.

This fastening tape produced as above would be slit to the desired size and then combined with one or two other tapes, e.g., a release tape and a target tape optionally together with fingerlift(s) and a centre stripe to form the laminate tape construction. A fingerlift facility may also be obtained by leaving the edge of paper, e.g., 1 to 6mm, uncoated with adhesive when the fastening tape is formed.

Examples of laminated tape constructions include:
1. A roll of tape comprising an elongate prelaminated tape composite wound convolutely upon itself about an annular core, especially suited for preparing a tape closure for disposable diapers by simply severing said elongate prelaminated tape composite parallel to the axis of the core at intervals corresponding to the predetermined width of said closure, the length of each such closure corresponding to the width of the roll of tape, said prelaminated tape composite comprising in combination:
   a. a fastening tape comprising an elongate strip of sheet backing material, having first and second edges, being substantially as wide as said tape composite and having a layer of a first normally tacky and pressure-sensitive adhesive coated over substantially one surface of said backing material.
   b. optionally, a layer of a second aggressive, normally tacky and pressure-sensitive adhesive coated over approximately one-third of the layer of said first normally tacky and pressure-sensitive adhesive along the first edge thereof.
   c. a fingerlift provided on the first pressure-sensitive adhesive adjacent the second edge thereof.
   d. a release tape, having first and second surfaces, the first surface adhered to said first pressure-sensitive adhesive layer.
   e. a layer of normally tacky and pressure-sensitive adhesive coated over the second surface of said release tape.
   f. a unifying strip centered along the junction of said second adhesive layer and the adhesive layer on said release tape and adhered to said adhesive layers, at least one of said tapes comprising a paper backing having a release coating of solventless, radiation-cured silicone applied directly to the paper. This construction is in accordance with U.S. Patent No. 4,726,971.
2. A roll of tape comprising an elongated prelaminated tape composite wound convolutely upon itself about an annular core, especially suited for preparing a tape closure for disposable diapers by simply severing said elongated prelaminated tape composite parallel to the axis of the core at intervals corresponding to the predetermined width of said closure, the length of each such closure corresponding to the width of the roll of tape, said prelaminated tape composite comprising in combination:
   a. a fastening tape divided into a bonded section and a fastening section with the fastening tape comprising an elongated strip of sheet backing material, having first and second edges, being substantially as wide as said tape composite, and having a first layer of normally tacky and pressure-sensitive adhesive coated over substantially one surface of said backing material.
   b. a first fingerlift provided on the first layer of pressure-sensitive adhesive adjacent the second edge thereof.
   c. a target tape, having first and second surfaces, the first surface adhered to said first pressure-sensitive adhesive layer.
   d. a second layer of normally tacky and pressuresensitive adhesive coated on the second surface of the target tape.
   e. a second fingerlift provided on the second layer of normally tacky and pressure-sensitive adhesive.
   f. a release tape having first and second surfaces, the first surface adhered to said second pressure-sensitive adhesive layer.
   g. a third layer of normally tacky and pressure-sensitive adhesive coated over the second surface of said release tape.
   h. a unifying strip centered over said release tape edge and adhered to the third adhesive layer and to the first adhesive layer on the fastening tape by folding under the target tape, at least one of said tapes comprising a paper backing having a release coating of solventless, radiation-cured silicone applied directly to the paper. This construction is in accordance with U.S. Patent No. 4,801,480.
3. A roll of tape as described in (2) with the exception that the unifying strip is not folded under the target tape.
4. A roll of tape comprising a composite elongate strip of pressure-sensitive adhesive sheet material wound convolutely upon itself about an annular core, especially suited for preparing tape strips to make the tape closure by simply severing said elongate strip of tape at right angles to the axis of the core at intervals corresponding to the predetermined width of said closure, the length of each such closure corresponding to width of the roll of tape, said composite elongate strip of tape characterized by comprising in combination:
   a. a fastening tape comprising an elongate strip of sheet backing material, having first and second edges, being substantially as wide as said composite strip, and having a layer of normally tacky and pressure-sensitive adhesive coated over at least substantially the entire width of said backing material.
   b. the adhesive of said fastening tape adjacent the first edge thereof contacting and adhered to the back of a pressure-sensitive adhesive target tape.
   c. the adhesive of said fastening tape adjacent the second edge thereof contacting and adhered to the back of a pressure-sensitive adhesive release tape, at least one of said tapes comprising a paper backing having a release coating of solventless, radiation-cured silicone applied directly to the paper. This construction is in accordance with European Patent No. 0148587.

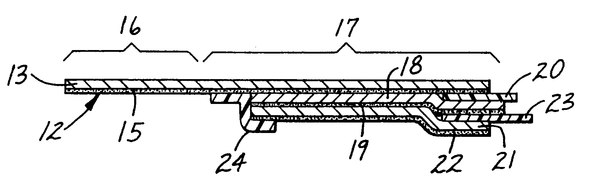

### Brief Description of the Drawing

The invention will now be described with reference to the accompanying drawing which represents a cross-section through a laminate tape construction in accordance with the invention.

### Description of the Preferred Embodiments

The composite tape is subdivided into bonded section (16) and fastening section (17) and made up of fastening tape (12), target tape (18), release tape (21) and unifying strip (24).

Fastening tape (12) comprises any suitable paper backing (13) provided a release coating of a solventless, radiation-cured silicone applied directly to the paper to facilitate unwinding of the composite tape when wound upon itself about the core. One face of the backing (13) is coated with a layer (15) of a tacky and aggressive pressure-sensitive adhesive. Suitable adhesives include conventional rubber-resin adhesives which have tack characteristics modified by the inclusion of tackifying resins such as the tackifying resins described in U.S. Patent No. 4,136,071. The aggressive pressure-sensitive adhesives used for layer (15) may also include conventional rubber-resin adhesives modified to have peel strengths between about 6 and 10 newtons per 25mm, preferably about 8 newtons per 25mm. A suitable method for measuring the peel strengths of adhesive layers on a steel, polyethylene or polypropylene surface is described in U.S. Patent No. 4,801,480.

Target tape (18), formed of any suitable tape backing material, is positioned so that it coincides with and covers part of adhesive layer (15). The top surface of target tape (18) is releasably adhered to adhesive layer (15). The bottom surface of target tape (18) is coated with a layer (19) of normally tacky and pressuresensitive adhesive. This adhesive layer (19) must form a strong shear bond to the outer surface of the diaper where it is adhered during use and may be the same as adhesive layer (15).

A first fingerlift (20) is positioned between fastening tape (12) and target tape (18). The first fingerlift (20) is adhered to fastening tape (12) by adhesive layer (15). Fingerlift (20) facilitates the lifting of fastening tape (12) from the target tape (18).

Release tape (21), formed of any suitable tape backing material, is positioned such that it substantially covers and is adhered to adhesive layer (19). The top surface of release tape (21) may be provided with a coating of release agent so that target tape (18) may be readily separated from release tape (21).

A second fingerlift (23) is adhesively attached to target tape (18) by adhesive layer (19). The fingerlift (23) is attached to an end portion of target tape (18) and facilitates the separation of target tape (18) from release tape (21) in order to allow initial positioning of target tape (18) and fastening tape (12) on the opposed side of the diaper.

Fingerlifts (20, 23) which are typically formed of narrow strips of polymeric film, are adhered to backing (13) and target tape (18) by adhesive layers (15, 19 respectively). The fingerlifts (20, 23) extend outwardly beyond the edge of fastening tape (12) and target tape (18) to permit and facilitate the separation of the various tapes. The separation of fastening tape (12) from target tape (18) is facilitated when it is desired to reopen the diaper closure.

Unifying strip (24), typically formed of a narrow strip of the same material as fingerlifts (20, 23) is positioned between end portions of tape such that its centerline coincides with the junction of target tape (18) and release tape (21) and adhesive layers (15, 19, 22). Thus, one part of unifying strip (24) is adhered to adhesive layer (22) and an approximately equal part is adhered to adhesive layer (15).

U.S. Patent No. 4,801,480 illustrates the use of closures formed by severing such a composite tape at intervals corresponding to the predetermined width of the closure, parallel to the axis of the tape core.

## Claims

1. A laminate tape construction comprising two or more tapes (12, 18 or 21) each having a layer of pressure-sensitive adhesive (15, 19 or 22) on a first face characterized in that at least one of the tapes (12, 18 or 21) comprises a porous paper backing (13, 18 or 21) having, on the opposite side to said adhesive layer, a continuous release coating of a solventless, radiation-cured silicone applied directly to the paper such that silicone is not on the porous paper backing (13, 18 or 21) first face.

2. A laminate tape construction comprising a fastening tape (12) divided into a bonded section (16) and a fastening section (17) with the fastening tape comprising an elongated strip of paper backing material (13), having first and second edges, being substantially as wide as said tape composite and having a first layer of normally tacky and pressure-sensitive adhesive (15) coated over substantially one surface of said backing material (13), and a release coating applied directly to the other surface, a target tape (18), having first and second surfaces, the first surface adhered to said first layer of pressure-sensitive adhesive (15); a second layer of normally tacky and pressure-sensitive adhesive (19) coated on the second surface of the target tape (18); a release tape (21), having first and second surfaces, the first surface adhered to said second layer of pressure-sensitive adhesive (19); a third layer of normally tacky and pressure-sensitive adhesive (22) coated over the second surface of said release tape (21), and a unifying strip (24) centered over said release tape (21) edge and adhered to the third layer of pressure-sensitive adhesives (22) and to the first adhesive layer (15) on the fastening tape characterized in that at least one of the tapes (12, 18 or 21) comprises a porous paper backing (13, 18 or 21) having on a first face said adhesive layer and on the opposite side to said adhesive layer, a continuous release coating of a solventless, radiation-cured silicone applied directly to the paper such that silicone is not on the porous paper backing (13, 18 or 21) first face.

3. A laminate tape construction as claimed in Claim 2 in which a first fingerlift (20) is interposed between the first adhesive layer (15) and the target tape (18) at the second edge and a second fingerlift (23) is interposed between the second adhesive layer (19) and the release tape (21) at the second edge.

4. A laminate tape construction as claimed in Claim 2 in which the paper backing (13) of the fastening tape (12) is free from adhesive at the second edge to provide a fingerlift facility.

5. A laminate tape construction as claimed in any preceding claim in which the porous paper backing has a basis weight of from 50 to 150 g/m².

6. A laminate tape construction as claimed in Claim 5 in which the porous paper backing has a basis weight of from 80 to 110 g/m².

7. A laminate tape construction as claimed in any preceding claim in which the coating weight of the solventless, radiation-cured silicone release coating is from 0.4 to 2.0 g/m².

8. A laminate tape construction as claimed in any preceding claim in which the pressure-sensitive adhesive layer (15, 19 or 22) is a hot melt adhesive or a solvent based adhesive.

9. A laminate tape as claimed in any preceding claim in which the peel strength of the pressure-sensitive adhesive layer (15, 19 or 22) on said solventless, radiation-cured silicone release layer is from 0.1 to 3.0 newtons per 25mm width.

10. A laminate tape as claimed in any preceding claim in which the porous paper backing is coloured and/or bears printed indicia.

## Patentansprüche

1. Laminatband-Aufbau, mit zwei oder mehreren Bändern (12,18 oder 21), die jeweils auf einer ersten Fläche eine Schicht aus Haftkleber (15,19 oder 22) aufweisen,
dadurch gekennzeichnet, daß mindestens eines der Bänder (12,18 oder 21) eine poröse Papier-Trägerschicht (13,18 oder 21) aufweist, die auf der der Kleberschicht entgegengesetzten Seite eine durchgehende Ablösebeschichtung aus lösungsmittelfreiem, strahlungsgehärteten Silicon aufweist, die direkt derart auf das Papier aufgetragen ist, daß sich kein Silicon auf der ersten Fläche der porösen Papier-Trägerschicht (13,18 oder 21) befindet.

2. Laminatband-Aufbau, mit einem Befestigungsband (12), das in einen Verbindungsabschnitt (16) und einen Befestigungsabschnitt (17) unterteilt ist, wobei das Befestigungsband aufweist: einen länglichen Streifen aus Papier-Trägermaterial (13), der erste und zweite Ränder aufweist, im wesentlichen die gleiche Breite wie der Band-Verbundkörper hat und eine erste Schicht aus normalerweise klebrigem und druckempfindlichen Haftkleber (15), die im wesentlichen auf eine Fläche des Trägermaterials (13) geschichtet ist, und eine Ablösebeschichtung aufweist, die direkt auf die andere Fläche aufgebracht ist, und ein Ziel-Band (18), das eine erste und eine zweite Fläche aufweist, wobei die erste Fläche an der ersten Schicht des Haftklebers (15) anhaftet; eine zweite Schicht aus normalerweise klebrigem und druckempfindlichen Haftkleber (19), die auf die zweite Fläche des Ziel-Bandes (18) geschichtet ist; ein Ablöseband (21) mit einer ersten und einer zweiten Fläche, wobei die erste Fläche an der zweiten Schicht aus Haftkleber (19) anhaftet; eine dritte Schicht aus normalerweise klebrigem und druckempfindlichen Haftkleber (22), die auf die zweite Fläche des Ablösebandes (21) geschichtet ist, und einen Vereinigungs-Streifen (24), der über dem Rand des Ablösebandes (21) zentriert ist und an der dritten Schicht aus Haftklebern (22) und an der ersten Kleberschicht (15) an dem Befestigungsband anhaftet,
dadurch gekennzeichnet, daß mindestens eines der Bänder (12,18 oder 21) eine poröse Papier-Trägerschicht (13,18 oder 21) aufweist, die auf einer ersten Fläche die Kleberschicht aufweist und auf der der Kleberschicht entgegengesetzten Seite eine durchgehende Ablösebeschichtung aus lösungsmittelfreiem, strahlungsgehärteten Silicon aufweist, die direkt derart auf das Papier aufgetragen ist, daß sich kein Silicon auf der ersten Fläche der porösen Papier-Trägerschicht (13,18 oder 21) befindet.

3. Laminatband-Aufbau nach Anspruch 2, bei der an dem zweiten Rand ein erstes Handabzugsende (20) zwischen der ersten Kleberschicht (15) und dem Ziel-Band (18) angeordnet ist und an dem zweiten Rand ein zweites Handabzugsende (23) zwischen der zweiten Kleberschicht (19) und dem Ablöseband (21) angeordnet ist.

4. Laminatband-Aufbau nach Anspruch 2, bei der zur Schaffung einer manuellen Abhebefunktion die Papier-Trägerschicht (13) des Befestigungsbandes (12) an dem zweiten Rand frei von Kleber ist.

5. Laminatband-Aufbau nach einem der vorhergehenden Ansprüche, bei der die poröse Papier-Trägerschicht ein Basisgewicht von 50 bis 150 g/m² aufweist.

6. Laminatband-Aufbau nach Anspruch 5, bei der die poröse Papier-Trägerschicht ein Basisgewicht von 80 bis 110 g/m² aufweist.

7. Laminatband-Aufbau nach einem der vorhergehenden Ansprüche, bei der das Beschichtungsgewicht der lösungsmittelfreiem, strahlungsgehärteten Silicon-Ablösebeschichtung von 0,4 bis 2,0 g/m² beträgt.

8. Laminatband-Aufbau nach einem der vorhergehenden Ansprüche, bei der die Haftkleberschicht (15,19 oder 22) ein Warmkleber oder ein Kleber auf Lösungsmittelbasis ist.

9. Laminatband-Aufbau nach einem der vorhergehenden Ansprüche, bei dem die Abzieh-Kraft der Haftkleberschicht (15,19 oder 22) auf der lösungsmittelfreiem, strahlungsgehärteten Silicon-Ablösebeschichtung von 0,1 bis 3,0 Newton pro 25 mm-Breite beträgt.

10. Laminatband-Aufbau nach einem der vorhergehenden Ansprüche, bei dem die poröse Papier-Trägerschicht gefärbt ist und/ oder gedruckte Markierungen trägt.

## Revendications

1. Structure de ruban stratifiée comprenant deux ou plus de deux rubans (12, 18 ou 21) comportant chacun une couche d'adhésif sensible à la pression (15, 19 ou 22) sur une première face, caractérisée en ce qu'au moins l'un des rubans (12, 18 ou 21) comprend un support de papier poreux (13, 18 ou 21) comportant, du côté opposé à la couche d'adhésif, un revêtement de séparation continu d'une silicone durcie par rayonnement, sans solvant appliqué directement au papier de telle sorte que la silicone ne soit pas sur la première face du support de papier poreux (13, 18 ou 21).

2. Structure de ruban stratifiée comprenant un ruban de fixation (12) divisé en une section liée (16) et une section de fixation (17), le ruban de fixation comprenant une bande allongée de matière de support de papier (13), comportant un premier et un second bord, ce ruban étant essentiellement aussi large que l'élément composite susdit en bande et comportant une première couche d'adhésif normalement collant et sensible à la pression (15) appliquée par-dessus essentiellement une surface de la matière de support (13), ainsi qu'un revêtement de séparation appliqué directement sur l'autre surface, un ruban cible (18), comportant une première et une seconde surface, la première surface étant amenée à adhérer à la première couche d'adhésif sensible à la pression (15), une seconde couche d'adhésif normalement collant et sensible à la pression (19) appliquée sur la seconde surface du ruban cible (18), un ruban de séparation (21), comportant une première et une seconde surface, la première surface étant amenée à adhérer à la seconde couche d'adhésif sensible à la pression (19), une troisième couche d'adhésif normalement collant et sensible à la pression (22) appliquée par-dessus la seconde surface du ruban de séparation (21) et une bande d'unification (24) centrée par-dessus le bord du ruban de séparation (21) et étant amenée à adhérer à la troisième couche d'adhésif sensible à la pression (22) et à la première couche d'adhésif (15) sur le ruban de fixation, caractérisée en ce qu'au moins l'un des rubans (12, 18 ou 21) comprend un support de papier poreux (13, 18 ou 21) comportant sur une première face la couche d'adhésif susdite et du côté opposé à cette couche d'adhésif, un revêtement de séparation continu d'une silicone durcie par rayonnement, sans solvant appliqué directement sur le papier de telle sorte que la silicone ne soit pas sur la première face du support de papier poreux (13, 18 ou 21).

3. Structure de ruban stratifiée suivant la revendication 2, dans laquelle un premier élément de soulèvement par les doigts (20) est intercalé entre la première couche d'adhésif (15) et le ruban cible (18) au second bord et un second élément de soulèvement par les doigts (23) est intercalé entre la seconde couche d'adhésif (19) et le ruban de séparation (21) à l'endroit du second bord.

4. Structure de ruban stratifiée suivant la revendication 2, dans laquelle le support de papier (13) du ruban de fixation (12) est exempt d'adhésif au second bord pour constituer un agencement de soulèvement par les doigts.

5. Structure de ruban stratifiée suivant l'une quelconque des revendications précédentes, dans laquelle le support de papier poreux a un poids de base de 50 à 150 g/m².

6. Structure de ruban stratifiée suivant la revendication 5, dans laquelle le support de papier poreux a un poids de base de 80 à 110 g/m².

7. Structure de ruban stratifiée suivant l'une quelconque des revendications précédentes, dans laquelle le poids de couche du revêtement de séparation de silicone durci au rayonnement, sans solvant est de 0,4 à 2,0 g/m².

8. Structure de ruban stratifiée suivant l'une quelconque des revendications précédentes, dans laquelle la couche d'adhésif sensible à la pression (15, 19 ou 22) est un adhésif fusible ou un adhésif à base de solvant.

9. Ruban stratifié suivant l'une quelconque des revendications précédentes, dans lequel la résistance au pelage de la couche d'adhésif sensible à la pression (15, 19 ou 22) sur la couche de séparation de silicone durcie par rayonnement, sans solvant est de 0,1 à 3,0 newtons par 25 mm de largeur.

10. Ruban stratifié suivant l'une quelconque des revendications précédentes, dans lequel le support de papier poreux est coloré et/ou porte des indices imprimés.
